# EUROPÄISCHE PATENTSCHRIFT

(11) **EP 0 712 849 B1**
(45) Veröffentlichungstag und Bekanntmachung des Hinweises auf die Patenterteilung: **17.01.2001**
(21) Anmeldenummer: 95117129.7
(22) Anmeldetag: 31.10.1995
(51) Int. Cl.: C07D 239/96

(54) **Verfahren zur Herstellung von substituierten Chinazolin-2,4-dionen**
Process of preparation of substituted quinazolin-2,4-diones
Procédé pour la préparation des quinazolin-2,4-diones substituées

(30) Priorität: 18.11.1994 DE 4441145
(43) Veröffentlichungstag der Anmeldung: 22.05.1996
(73) Patentinhaber: Clariant GmbH, 65929 Frankfurt am Main (DE)
(72) Erfinder: Cosmo, Robert, Dr., D-64291 Darmstadt (DE)

(56) Entgegenhaltungen:
- EP-A- 0 184 258
- EP-A- 0 545 206
- EP-A- 0 653 423
- DE-A- 3 712 782
- CHEMICAL ABSTRACTS, vol. 83, no. 11, 15.September 1975 Columbus, Ohio, US; abstract no. 97348u, Seite 596; Spalte 2; XP002003562 & JP-A-50 014 689 (RESEARCH INSTITUTE FOR PRODUCTION DEVELOPMENT) 15.Februar 1975

## Beschreibung

Substituierte Chinazolin-2,4-dione der allgemeinen Formel (I) sind interessante Zwischenprodukte für Pharmaceutiza und Pflanzenschutzmittel (US PS 4.405.623; GB 1.059.271; EP 360.417).

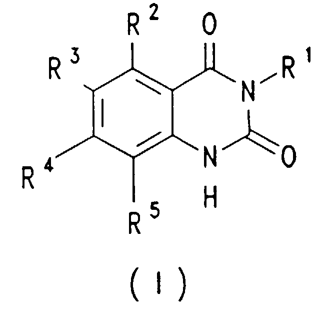

Die Herstellung von (I) erfolgt üblicherweise in zwei Reaktionsschritten:

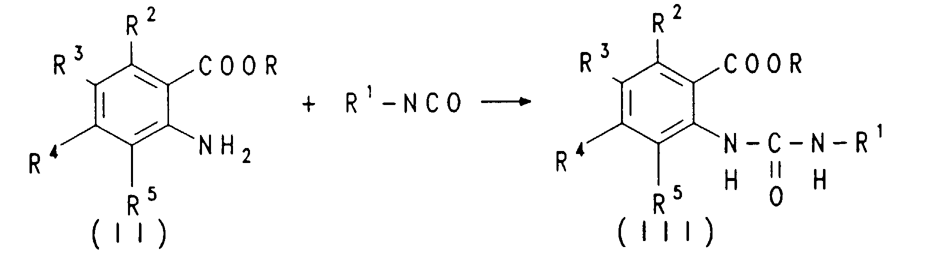

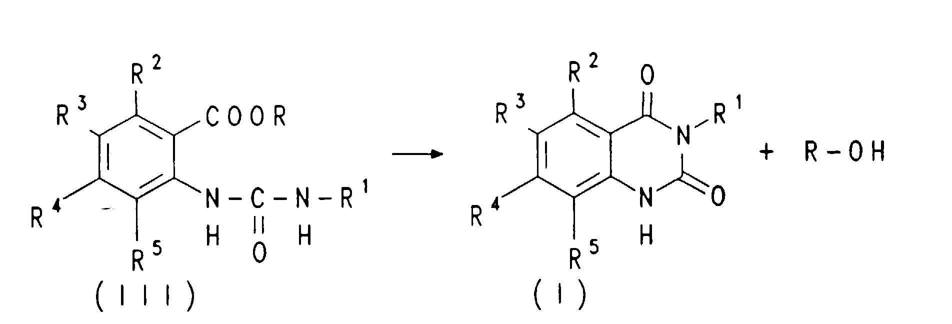

In erster Stufe wird durch Umsetzung einer - gegebenenfalls substituierten - Anthranilsäure oder eines Anthranilsäurealkylesters der allgemeinen Formel (II) mit einem Isocyanat in einem, gegenüber Isocyanaten inerten Reaktionsmedium die Zwischenstufe (III) erhalten.

Im Falle von Anthranilsäuren erhält man so N-Alkylcarbamoylanthranilsäuren (III, R = H), die zwischenisoliert werden und dann in einem zweiten Reaktionsschritt, z.B. in 10 % wäßriger Schwefelsäure in Gegenwart von einigen Tropfen 95 % Ethanol [J. Org. Chem., 18, 1427 (1953)] zu Chinazolindionen der allgemeinen Formel (I) ringgeschlossen werden. Im Falle von N-Cycloalkylcarbamoylanthranilsäuren (III), R = Cycloalkyl) wurde bereits beschrieben, daß die Cyclisierung zu (I) in einem protischen organischen Medium, wie z.B. Ethanol in Gegenwart überschüssiger starker Mineralsäure, wie z.B. konzentrierter Schwefelsäure, durchführbar ist (DOS 1.542.824).

In entsprechender Weise liefern die Anthranilsäurealkylester bei der Umsetzung mit Isocyanaten N-Alkylcarbamoylanthranilsäurealkylester (III, (R = Alkyl) die zwischenisoliert und in analoger Weise zu der oben beschriebenen Cyclisierung der N-Cycloalkylcarbamoylanthranilsäuren (III), R = Cycloalkyl) ringgeschlossen werden [GB 2.108.495, J. Organic Chemistry, 26, S. 5238 (1961)]. Für die Verbindungen III (R = Alkyl, Aralkyl) ist es auch bekannt, die Cyclisierung zu (I), in protischen Medien wie z.B. Ethanol oder Methanol in Gegenwart von wäßrigen Natriumhydroxid durchzuführen [DOS 1.804.391, J. Heterocycl. Chem. 19 (2), S. 269 (1982)].

Diese Herstellverfahren für substituierte Chinazolin-2,4-dione haben jedoch den wesentlichen Nachteil, daß die Herstellung der Zwischenstufe (III) stets in einem anderen Reaktionsmedium erfolgt als die Cyclisierung von (III) zu (I).

Dieses Tatsache beruht offensichtlich auf dem Vorurteil, daß der Ringschluß von (III) zu (I) nur in protischen, stark polaren Medien durchgeführt werden kann, wie z.B. in Alkoholen, starken Mineralsäuren oder alkoholisch-wäßrig alkalischen Medien. Diese Medien sind jedoch ihrerseits erwiesenermaßen für die Herstellung von (III) aus (II) und Arylisocyanaten ungeeignet, da sie mit Isocyanaten selbst äußerst heftig reagieren. Dieser Umstand führt somit zu der Verwendung unterschiedlicher Reaktionsmedium für die Herstellung von (III) und (I) und damit zwangsläufig zu erhöhtem technischem und wirtschaftlichem Aufwand, der mit einem Wechsel des Reaktionsmediums und einer Produktzwischenisolierung naturgemäß verbunden ist.

In Europ. J. Med. Chem., 9, S. 263 (1974) wurde beschrieben, daß die Umsetzung von Anthranilsäuremethylester (II, R = CH₃, R², R³, R⁴, R⁵=H) mit Alkyl- oder Arylisocyanaten zum N-substituierten Carbamoylanthranilsäuremethylester (III, R = CH₃) und die anschließende Cyclisierung zu Chinazolin-2,4-dionen (I) in demselben Reaktionsmedium (Benzol oder Toluol) durchführbar ist. Dabei werden unterstöchiometrische Mengen Triethylamin als Cyclisierungskatalysator eingesetzt. Die Reaktion kann auch ohne Lösungsmittel ablaufen. Dieses Verfahren hat jedoch den Nachteil, daß hohe Reaktionstemperaturen bzw. lange Reaktionszeiten notwendig sind, um das Zwischenprodukt N-Aryl- bzw. N-Alkylcarbamoylanthranilsäurealkylester (III, R=alkyl) in das Chinazolin-2,4-dion (I) zu überführen. Die Produkte dieses Verfahrens sind im allgemeinen mit dem Zwischenprodukt N-Aryl- bzw. Alkylcarbamoylanthranilsäurealkylester (III, R=alkyl) verunreinigt. Nur durch Anwendung von überschüssigem Triethylamin kann im allgemeinen eine vollständige Cyclisierung erzielt werden.

Daß das Erhitzen von Anthranilsäuremethylester mit Alkylisocyanaten in Gegenwart von Triethylamin lediglich zur Bildung des entsprechenden N-Alkylcarbamoylanthranilsäuremethylester führt ist auch der GB 2.108.495 und J. Org. Chem. 26, 5238 (1961) zu entnehmen. Hier wird die Cyclisierung zu den gewünschten Chinazolin-2,4-dionen erst durch Erhitzen dieser Zwischenstufe in konzentrierter Salzsäure und Ethanol erreicht.

Die bereits erwähnte Cyclisierung von (III) mit R=Alkyl; zu (I) in Methanol oder Ethanol als Reaktionsmedium und mit einer überstöchiometrischen Menge an ca. 5 bis 10 %igem wäßrigem Natriumhydroxid (DE 1.804.391); J. Heterocycl. Chem. 19 (2) S. 269 (1982) hat trotz sehr kurzer Reaktionszeiten und guter Ausbeuten den Nachteil, daß dabei zunächst das Natriumsalz des Chinazolin-2,4-dion erhalten wird, welches anschließend durch Behandlung mit einer Mineralsäure bzw. einer organischen Säure in das freie Dion überführt werden muß, wobei 1 Mol unerwünschtes, abwasserbelastendes Natriumsalz der Mineralsäure bzw. der organischen Säure entsteht.

EP-A1-0 545 206 offenbart ein Verfahren zur Herstellung von N-Aryl-Stickstoffheterocyclen, insbesondere Pyrimidin-2,4-dione, wobei ein Crotonsäureester mit einem Isocyanat in Gegenwart eines Überschusses an Base umgesetzt wird, wobei nur mäßige Ausbeuten erzielt werden. In der JP-A-50 014 689 wird ebenfalls ein Überschuß an Base bei der Umsetzung von Anthranilsäureestern mit Isocyanaten verwendet.

In der EP-A1-0 184258 werden Isocyanatobenzoate mit Aminen in Gegenwart einer Base zu Chinazolindionen cyclisiert.

In der DE-A- 37 12 782 werden Enaminoester mit Isocyanaten unter alkalischen Bedingungen zu Harnstoffderivaten cyclisiert.

In der EP-A1-0 653 423 werden schließlich N-Aryl-chinazolin-2,4-dione durch Umsetzug von Anthranilsäureestern mit Arylisocyanaten in einem aprotischen Reaktionsmedium und anschließender Cyclisierung mit einer Base erhalten.

Es bestand somit ein großer Bedarf nach einem Verfahren, das es erlaubt, substituierte Chinazolin-2,4-dione in hoher Ausbeute und Reinheit auf technisch einfache Weise bei geringer Umweltbelastung zu erhalten.

Diese Aufgabe wird gelöst durch ein Verfahren zur Herstellung von Chinazolin-2,4-dionen der allgemeinen Formel (I) 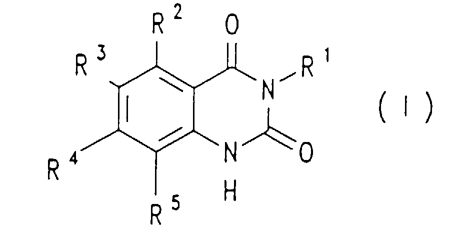 worin R¹ (C₁-C₁₂)Alkyl, (C₃-C₈)Cycloalkyl, (C₇-C₁₀)Aralkyl, wobei die Arlygruppe durch ein oder mehrere Halogenatome substituiert sein kann und R², R³, R⁴, R⁵ unabhängig voneinander Halogen, C₁-C₃-Alkyl, C₁-C₃-Alkoxy oder Wasserstoff bedeuten, ausgehend von einem Isocyanat R¹-N=C=O und einem Anthranilsäureester der allgemeinen Formel (II) 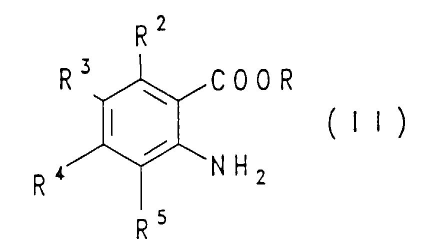 worin R gleich (C₁-C₁₀)Alkyl bedeutet und R² bis R⁵ die oben angegebene Bedeutung besitzen, dadurch gekennzeichnet, daß man den Anthranilsäureester in einem aprotischen Reaktionsmedium mit dem Isocyanat R¹-N = C = O zu Carbamoylanthranilsäureestern der allgemeinen Formel (III) 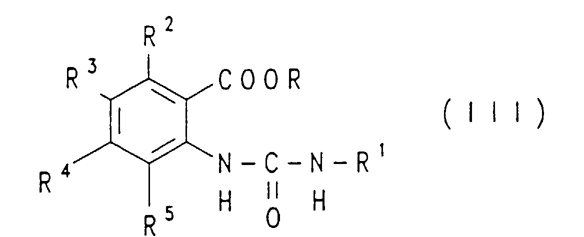 worin R, R¹ bis R⁵ die oben angegebene Bedeutung besitzen, umsetzt und diese in Gegenwart von Alkali- oder Erdalkalimetallakoholaten, -amiden, -hydriden oder Tetraalkylammoniumhydroxiden cyclisiert.

Wichtig ist das Verfahren z.B. zur Herstellung von Verbindungen der Formel (I) worin R¹ gleich (C₁-C₆)Alkyl, (C₃-C₆)Cycloalkyl oder (C₇-C₈)Aralkyl, wobei die Aryl-Gruppe der (C₇-C₈)Aralkyl-Gruppe durch eines oder mehrere Halogenatome substituiert sein kann, ist, R gleich (C₁-C₃)-Alkyl bedeutet und R² bis R⁵ unabhängig voneinander Wasserstoff, (C₁-C₃)-Alkyl, (C₁-C₃)-Alkoxy, Fluor, Chlor oder Brom bedeuten. Hierbei sind wiederum die Verbindungen wichtig worin R² bis R⁵ unabhängig voneinander Wasserstoff, Fluor, Chlor oder Methyl bedeuten.

Von besonderem Interesse ist die Umsetzung von 4-Chloranthranilsäurealkylester (A) mit 4-Brom-2-fluorbenzylisocyanat (B) zu N-(4-Brom-2-fluorphenylcarbamoyl)-4-chloranthranilsäurealkylester (C) und anschließende Cyclisierung zu 3-(4-Brom-2-fluorbenzyl)-7-chlor-2,4(1H,3H)-chinazolindion (D) nach dem erfindungsgemäßen Verfahren. 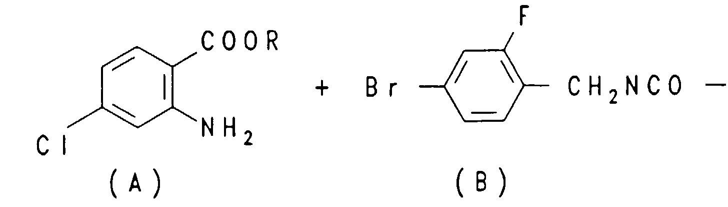 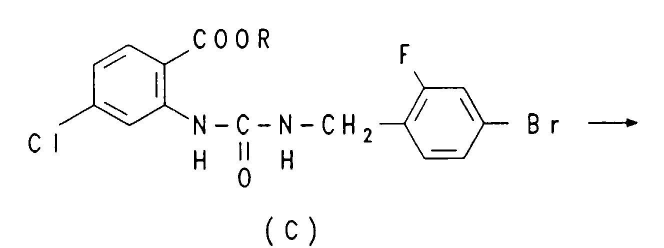 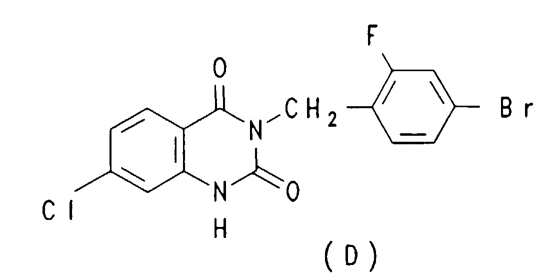 4-Brom-2-fluorbenzylisocyanat (B) kann durch Umsetzung von 4-Brom-2-fluor-benzylamin oder seines Hydrochlorids mit Phosgen in einem inerten Reaktionsmedium in hoher Ausbeute hergestellt werden.

Die Herstellung von (D) ist bereits in der EP 218 999 beschrieben:
Hierbei wird in der ersten Stufe 7-Chlor-2H-3,1-benzoxaxin-2,4(1H)-dion mit 4-Brom-2-fluorbenzylamin in THF als Reaktionsmedium zu 2-Amino-N-(4-brom-2-fluorbenzyl)-4-chlorbenzamid umgesetzt (Seite 16, Preparation 11) welches in der zweiten Stufe durch die Carbonylierung mit N,N'-Carbonyldiimidazol in Dioxan zu (D) umgewandelt wird (Seite 19, Preparation 16, Nr. 4).

Das in EP 218.999 beschriebene Verfahren zur Herstellung von (D) hat erhebliche wirtschaftliche Nachteile: Bei der ersten Stufe wird ein Überschuß (37 Mol-%) vom teuren 4-Brom-2-fluorbenzylamin bezogen auf den Reaktionspartner 7-Chlor-2H-3,1-benzoxazin-2,4(1H)-dion eingesetzt. Die isolierte Ausbeute bezogen auf 4-Brom-2-fluorbenzylamin beträgt nur 59 %. In der zweiten Stufe, der Umsetzung zum Chinazolindion-Derivat (D) wird das teure Reagenz N,N'-Carbonyldiimidazol im sehr großen Überschuß (300 Mol-%) eingesetzt.

Gegenüber dem in EP 218.999 beschriebenen Verfahren zur Herstellung von (D) hat das erfindungsgemäße Verfahren folgende Vorteile: Die Ausbeuten der beiden Stufen, der Phosgenierung des Hydrochlorids zu (B) und der Bildung von (D) aus (A) und (B), sind hoch. Die Gesamtausbeute an (D) bezogen auf das teure 4-Brom-2-fluorbenzylamin liegt daher deutlich höher (79 % d. Th.) als bei dem in EP 218.999 beschriebenen Verfahren. Außerdem werden keine teuren Reagenzien wie N,N'-Carbonyldiimidazol benötigt. Somit ist das erfindungsgemäße Verfahren erheblich kostengünstiger.

Die Cyclisierung von (III) zu (I) verläuft mit hervorragenden Ausbeuten in aprotischen Reaktionsmedien, die als solche inert gegenüber Isocyanaten sind. Bevorzugt werden dieselben Reaktionsmedien verwendet, in welchen auch die Verbindung (III) hergestellt wird, wie z.B. aromatische, aliphatische oder cycloaliphattische Kohlenwasserstoffe, Heterocyclen oder Ketone, insbesondere einkernige Alkylaromaten oder, bei Normalbedingungen flüssige Alkane und Cycloalkane. In vielen Fällen haben sich Toluol und Xylole in reiner Form oder als Isomerengemisch bewährt.

Damit besteht die vorteilhafte Möglichkeit, die Umsetzung von (II) mit dem Isocyanat zu (III) und die Cyclisierung von (III) zu (I) in einem und demselben Reaktionsmedium bei niedrigen Temperaturen mit sehr kurzen Reaktionszeiten durchzuführen; hierbei kann auf eine Zwischenisolierung von (III) verzichtet werden. Jedoch ist es auch möglich (III), z.B. aus Gründen einer zusätzlichen Reinigung, zu isolieren und anschließend erneut in frischem Reaktionsmedium zu (I) zu cyclisieren.

Als Anthranilsäureester werden im allgemeinen die Alkylester eingesetzt; bevorzugt kommen substituierte Anthranilsäuremethylester, insbesondere halogensubstituierte Anthranilsäuremethylester zum Einsatz.

Die als Base verwendeten Alkali- oder Erdalkalimetallalkoholate können in Substanz oder als Lösungen in den entsprechenden Alkoholen eingesetzt werden. In vielen Fällen hat sich der Einsatz von Natriummethylat in Methanol bewährt.

Verwendet man Alkali- oder Erdalkalimetallhydride oder Amide, so können diese ebenfalls in Substanz oder als Suspension im aprotischen Solvent eingesetzt werden.

Die verwendeten Basen werden üblicherweise in Mengen von 1 bis 95 Mol-% bezogen auf die Carbamoylanthranilsäureester (III) eingesetzt. Es hat sich als günstig erwiesen, Mengen von 1 bis 20 Mol-%, insbesondere 5 bis 10 Mol-% zu verwenden.

Das in Europ. J. Med. Chem. 9, S. 263 (1974) beschriebene Verfahren konnte, wie die Beispiele 6 und 7 zeigen für Verbindungen mit R¹ gleich Propyl oder Benzyl nicht nachgearbeitet werden. Mit Triethylamin als Base wurden nur die N-Propyl- bzw. N-Benzyl-carbamoylanthranilsäureester erhalten. Weiterreaktion zu den Chinazolindionen fand nicht statt. Im Gegensatz hierzu führte die Reaktion mit Natriummethylat in hohen Ausbeuten zu den gewünschten Verbindungen (Beispiele 4 und 5).

Die Reaktionstemperatur für die Umsetzung des Anthranilsäureethylester mit dem Isocyanat liegt im allgemeinen zwischen ca. 20°C und dem Siedepunkt des verwendeten Reaktionsmediums, bevorzugt in einem Bereich zwischen 30 und 120°C. Die Reaktionstemperatur der Cyclisierung (III) zu (I) wird zweckmäßig so gewählt, daß der freiwerdende Alkohol aus dem Reaktionsmedium abdestilliert; im Falle des bevorzugten Methyl- oder Ethylesters von (II) entspricht dies einer Reaktionstemperatur oberhalb des Siedepunktes von Methanol bzw. Ethanol. Die Abdestillation des freiwerdenden Alkohols aus dem Reaktionsgemisch ist jedoch nicht entscheidend für das Gelingen der Cyclisierung von (III) zu (I). Die Cyclisierungsreaktion verläuft ebensogut unter Rückfluß des freiwerdenden Alkohols.

### Beispiele

### Beispiel 1: Herstellung von 4-Brom-2-fluorbenzylisocyanat durch Phosgenierung von 4-Brom-2-fluorbenzylamin

Chlorbenzol (30 ml) wird bei 60°C vorgelegt und mit Phosgen gesättigt. Eine Lösung von 4-Brom-2-flourbenzylamin (20,4 g, 0,10 Mol) in Chlorbenzol (40 ml) wird in 3 Stunden bei 60°C zugetropft. Gleichzeitig wird Phosgen eingeleitet. Der Ansatz wird 2 Stunden bei 60°C nachgerührt und anschließend zwecks Entgasen von Phosgen und Chlorwassersoff zum Rückfluß erhitzt. Der Ansatz wird destillativ aufgearbeitet. Es werden 15,9 g 4-Brom-2-fluorbenzylisocyanat (Siedepunkt 109°C bei 4 mbar) erhalten. Ausbeute: 69,1 % d.Th.

### Beispiel 2: Herstellung von 4-Brom-2-fluorbenzylisocyanat durch Phosgenierung von 4-Brom-2-fluorbenzylamin-Hydrochlorid

Eine Lösung von 4-Brom-2-fluorbenzylamin (20,4 g, 0,10 Mol) in Toluol (300 ml) wird bei 25°C mit gasförmiger Salzsäure gesättigt. Die daraus gebildete Suspension des 4-Brom-2-fluorbenzylamin-hydrochlorids wird auf 100°C erhitzt. Bei dieser Temperatur wird Phosgen eingeleitet. Die schließlich klar gewordene Lösung wird 1 Stunde bei 100°C nachgerührt und anschließend zwecks Entgasen von Phosgen und Chlorwasserstoff zum Rückfluß erhitzt. Der Ansatz wird destillativ aufgearbeitet. Es werden 21,2 g 4-Brom-2-fluorbenzylisocyanat (Siedepunkt 109°C bei 4 mbar) erhalten. Ausbeute: 92,1 % d. Th.

### Beispiel 3: Herstellung von 3-(4-Brom-2-fluorbenzyl)-7-chlor-2,4(1H,3H)-chinazolindion

In einer Lösung von 4-Chloranthranilsäuremethylester (37,1 g, 0,20 Mol) in Xylol (250 ml) wird eine Lösung von 4-Brom-2-fluorbenzylisocyanat (46,0 g, 0,20 Mol) in Xylol (100 ml) bei Raumtemperatur zudosiert. Nach beendeter Zugabe wird der Ansatz 2 Stunden bei 110°C gerührt. Es werden 3,60 ml einer 30 %igen Lösung von methanolischem Natriummethylat (0,020 Mol) zudosiert und 2 Stunden bei 90°C gerührt, wobei Methanol abdestilliert wird. Anschließend werden 1,8 ml 85 % Ameisensäure (0,040 Mol) zugegeben und weitere 1 Stunde bei 90°C gerührt. Nach Abkühlung wird das Produkt abgesaugt, mit 3 x 50 ml Xylol, 3 x 50 ml Ethanol, 3 x 100 ml Wasser gewaschen und getrocknet. Es werden 66,1 g 3-(4-Brom-2-fluorbenzyl)-7-chlor-2,4(1H,3H)-chinazolindion (Ausbeute: 86,2 % d. Th.) mit Schmelzpunkt 288 bis 289°C (lit. > 280°C) erhalten.

### Beispiel 4: Herstellung von 3-(Benzyl)-2,4(1H,3H)-chinazolindion

In einer Lösung von Anthranilsäuremethylester (15,1 g, 0,10 mol) in Xylol (80 ml) wird eine Lösung von Benzylisocyanat (13,3 g, 0,10 mol) in Xylol (40 ml) bei Raumtemperatur zudosiert. Nach beendeter Zugabe wird der Ansatz 2 Stunden bei 110°C gerührt. Es werden 1,80 ml einer 30 %igen Lösung von methanolischem Natriummethylat (0,010 ml) zudosiert und weitere 2 Stunden bei 90°C gerührt, wobei Methanol abdestilliert wird. Anschließend werden 0,9 ml 85 % Ameisensäure (0,020 mol) zugegeben und weitere 1 Stunde bei 90°C gerührt. Nach Abkühlung wird das Produkt abgesaugt, mit 3 x 25 ml Xylol, 3 x 25 ml Ethanol, 3 x 25 ml Wasser gewaschen und getrocknet. Es werden 22,2 g 3-(Benzyl)-2,4(1H,3H)-chinazolindion (Ausbeute: 88,0 % d. Th.) mit Schmelzpunkt 231 bis 232°C (lit. 227 bis 228°C) erhalten.

### Beispiel 5: Herstellung von 3-(Propyl)-2,4(1H,3H)-chinazolindion

In einer Lösung von Anthranilsäureethylester (66,1 g, 0,40 mol) in Xylol (200 ml) wird eine Lösung von Propylisocyanat (34,0 g, 0,40 mol) in Xylol (100 ml) bei Raumtemperatur zudosiert. Nach beendeter Zugabe wird der Ansatz 2 Stunden bei 110°C gerührt. Es werden 7,20 ml einer 30 %igen Lösung von methanolischem Natriummethylat (0,040 ml) zudosiert und weitere 2 Stunden bei 90°C gerührt, wobei Methanol und Ethanol abdestilliert werden. Anschließend werden 3,6 ml 85 % Ameisensäure (0,080 mol) zugegeben und weitere 1 Stunde bei 90°C gerührt. Nach Abkühlung wird das Produkt abgesaugt, mit 3 x 25 ml Xylol, 3 x 25 ml Ethanol, 3 x 25 ml Wasser gewaschen und getrocknet. Es werden 67,3 g 3-(Propyl)-2,4(1H,3H)-chinazolindion (Ausbeute: 82,4 % d. Th.) mit Schmelzpunkt 190 bis 191°C (lit. 187 bis 188°C) erhalten.

### Beispiel 6: Versuch zur Herstellung von 3-(Benzyl)-2,4(1H,3H)-chinazolindion (Vergleichsbeispiel Katalysator: Triethylamin) - Analog zum Beispiel 4

In einer Lösung von Anthranilsäuremethylester (15,1 g, 0,10 mol) in Xylol (80 ml) wird eine Lösung von Benzylisocyanat (13,3 g, 0,10 mol) in Xylol (40 ml) bei Raumtemperatur zudosiert. Nach beendeter Zugabe wird der Ansatz 2 Stunden bei 110°C gerührt. Triethylamin (1,4 ml, 1,01 g, 0,010 mol) wird zugegeben. Es werden weitere 2 Stunden bei 90°C gerührt. Das gewünschte Cyclisierungsprodukt 3-(Benzyl)-2,4(1H,3H)-chinazolindion ist in der Reaktionslösung durch Dünnschichtchromatographie nicht nachweisbar. Nach Abkühlung wird der Niederschlag abgesaugt, mit 3 x 25 ml Xylol gewaschen und getrocknet. Es werden 23,1 g N-(Benzylcarbamoyl)anthranilsäuremethylester (Ausbeute: 81,3 % d. Th.) mit Schmelzpunkt 140 bis 141°C (lit. 139 bis 140°C) erhalten.

### Beispiel 7: Versuch zur Herstellung von 3-(Propyl)-2,4(1H,3H)-chinazolindion (Vergleichsbeispiel Katalysator: Triethylamin) - Analog zum Beispiel 5

In einer Lösung von Anthranilsäureethylester (66,1 g, 0,40 mol) in Xylol (200 ml) wird eine Lösung von Propylisocyanat (34,0 g, 0,40 mol) in Xylol (100 ml) bei Raumtemperatur zudosiert. Nach beendeter Zugabe wird der Ansatz 2 Stunden bei 110°C gerührt. Triethylamin (5,6 ml, 4,08 g, 0,040 mol) wird zugegeben. Es wird weitere 2 Stunden bei 90°C gerührt. Das gewünschte Cyclisierungsprodukt 3-(Propyl)-2,4(1H,3H)-chinazolindion ist in der Reaktionslösung durch Dünnschichtchromatographie nicht nachweisbar. Nach Abkühlung wird der Niederschlag abgesaugt, mit 3 x 25 ml Xylol gewaschen und getrocknet. Es werden 67,0 g N-(Propylcarbamoyl)anthranilsäureethylester (Ausbeute: 66,9 % d. Th.) mit Schmelzpunkt 98 bis 99°C erhalten.

## Patentansprüche

1. Verfahren zur Herstellung von Chinazolin-2,4-dionen der allgemeinen Formel (I) 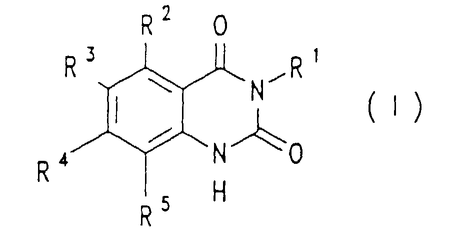 worin R¹ (C₁-C₁₂)Alkyl, (C₃-C₈)Cycloalkyl, (C₇-C₁₀)Aralkyl, ist wobei die Arylgruppe durch ein oder mehrere Halogenatome substituiert sein kann und R², R³, R⁴, R⁵ unabhängig voneinander Halogen, C₁-C₃-Alkyl, C₁-C₃-Alkoxy oder Wasserstoff bedeuten, ausgehend von einem Isocyanat R¹-N = C = O und einem Anthranilsäureester der allgemeinen Formel (II) 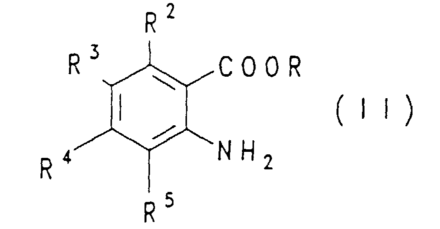 worin R gleich (C₁-C₁₀)Alkyl bedeutet und R² bis R⁵ die oben angegebene Bedeutung besitzen, dadurch gekennzeichnet, daß man den Anthranilsäureester in einem aprotischen Reaktionsmedium mit dem Isocyanat zu Carbamoylanthranilsäureestern der allgemeinen Formel (III) 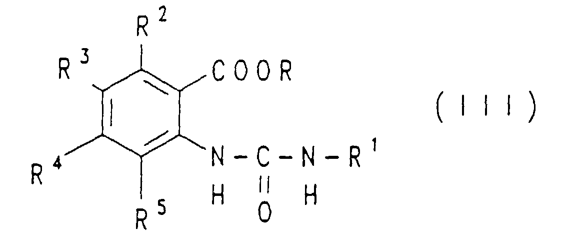 worin R, R¹ bis R⁵ die oben angegebene Bedeutung besitzen, umsetzt und diese in Gegenwart von Alkali- oder Erdalkalimetallakoholaten, -amiden, -hydriden oder Tetraalkylammoniumhydroxiden cyclisiert.

2. Verfahren nach Anspruch 1, dadurch gekennzeichnet, daß R¹ gleich (C₁-C₆)Alkyl oder (C₃-C₆)Cycloalkyl oder (C₇-C₈)Aralkyl, wobei die Aryl-Gruppe der (C₇-C₈)Aralkyl-Gruppe durch eines oder mehrere Halogenatome substituiert sein kann, R gleich (C₁-C₃)-Alkyl ist und R² bis R⁵ unabhängig voneinander Wasserstoff, (C₁-C₃)-Alkyl, (C₁-C₃)-Alkoxy, Fluor, Chlor oder Brom, insbesondere Wasserstoff, Fluor, Chlor oder Methyl bedeuten.

3. Verfahren nach Anspruch 1, dadurch gekennzeichnet, daß 4-Chloranthranilsäure-alkylester (A) mit 4-Brom-2-fluorbenzylisocyanat (B) zu N-(4-Brom-2-fluorphenylcarbamoyl)-4-chloranthranilsäurealkylester (C) und 3-(4-Brom-2-fluorbenzyl)-7-chlor-2,4(1H,3H)-chinazolindion (D) umgesetzt wird.

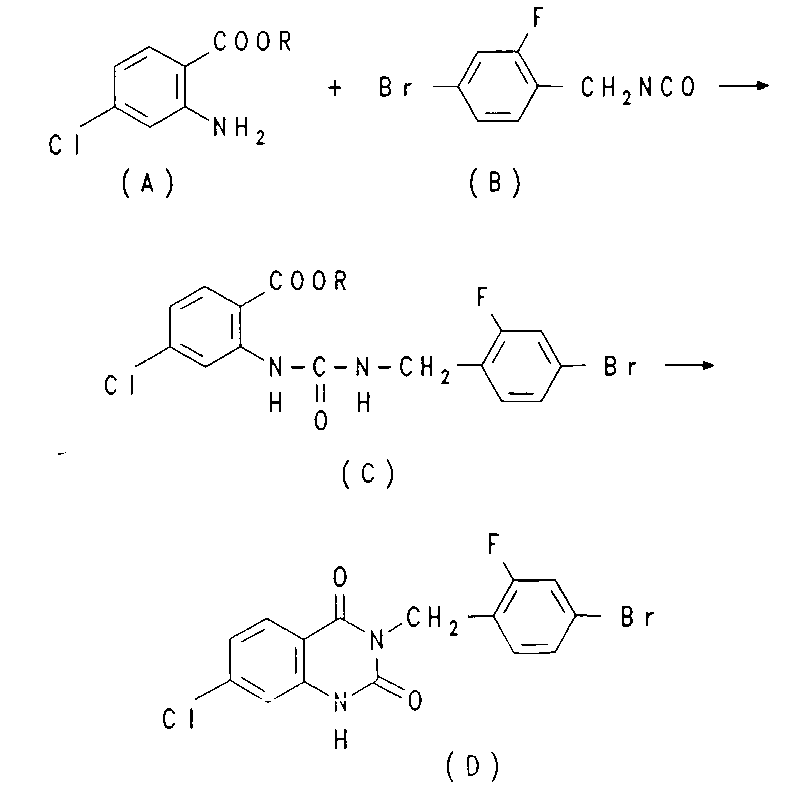

4. Verfahren nach mindestens einem der Ansprüche 1 bis 3, dadurch gekennzeichnet, daß man als aprotisches Reaktionsmedium aromatische, cycloaliphatische oder aliphatische Kohlenwasserstoffe, Heterocyclen oder Ketone einsetzt.

5. Verfahren nach mindestens einem der Ansprüche 1 bis 3, dadurch gekennzeichnet, daß man als aprotisches Reaktionsmedium einen aromatischen Kohlenwasserstoff, insbesondere ein Alkylbenzol, bevorzugt Xylol oder deren Isomerengemisch verwendet.

6. Verfahren nach mindestens einem der Ansprüche 1 bis 5, dadurch gekennzeichnet, daß man die Alkali- oder Erdalkalimetallalkoholate, -amide, -hydride oder Tetraalkylammoniumhydroxide in Mengen von 1 bis 95 Mol-%, insbesondere 1 bis 20 Mol-%, bevorzugt 5 bis 10 Mol-% bezogen auf (III) einsetzt.

7. Verfahren nach mindestens einem der Ansprüche 1 bis 6, dadurch gekennzeichnet, daß als Alkoholat Natriummethylat in Methanol eingesetzt wird.

8. Verfahren nach mindestens einem der Ansprüche 1 bis 7, dadurch gekennzeichnet, daß die Umsetzung des Isocyanats mit dem Anthranilsäureester bei Temperaturen von 20 bis 150°C, insbesondere 30 bis 120°C durchgeführt wird.

9. 4-Brom-2-fluorbenzylisocyanat

## Claims

1. A process for the preparation of quinazoline-2,4-diones of the formula (I) 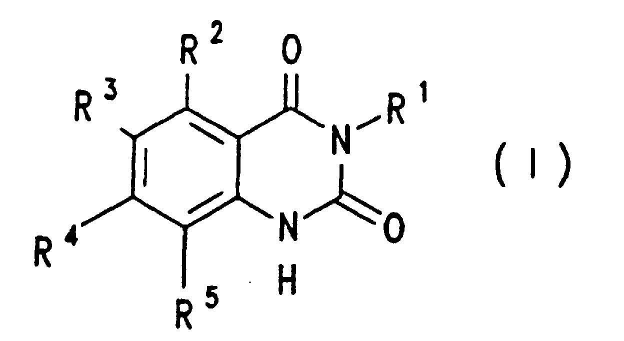 in which R¹ is (C₁-C₁₂)alkyl, (C₃-C₈)cycloalkyl or (C₇-C₁₀)aralkyl, it being possible for the aryl group to be substituted by one or more halogen atoms, and R², R³, , R⁴ and R⁵, independently of one another, are halogen, C₁-C₃-alkyl, C₁-C₃-alkoxy or hydrogen, starting from an isocyanate R¹-N=C=0 and an alkoxy anthranilic acid ester of the formula (II) 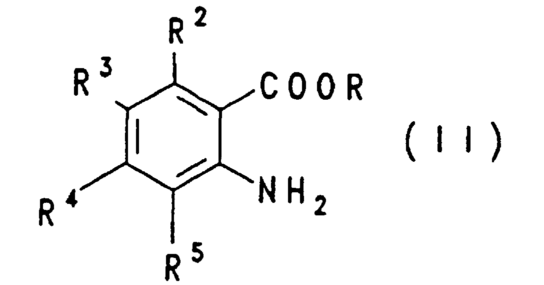 in which R is (C₁-C₁₀)alkyl and R² to R⁵ are as defined above, which comprises reacting the anthranilic acid ester with the isocyanate in an aprotic reaction medium to give carbamoylanthranilic acid esters of the formula (III) 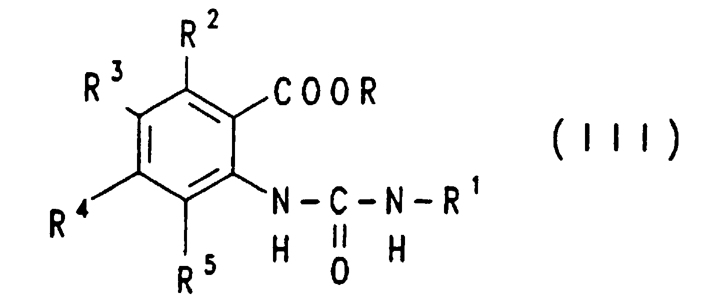 in which R, R¹ to R⁵ are as defined above, and cyclizing these products in the presence of alkali metal alcoholates, alkali metal amides, alkali metal hydrides, alkaline earth metal alcoholates, alkaline earth metal amides, alkaline earth metal hydrides or tetraalkylammonium hydroxides.

2. The process as claimed in claim 1, wherein R¹ is (C₁-C₆)alkyl, (C₃-C₆)cycloalkyl or (C₇-C₈)aralkyl, it being possible for the aryl group of the (C₇-C₈)aralkyl group to be substituted by one or more halogen atoms, R is (C₁-C₃)alkyl and R² to R⁵, independently of one another, are hydrogen, (C₁-C₃)alkyl, (C₁-C₃)alkoxy, fluorine, chlorine or bromine, in particular hydrogen, fluorine, chlorine or methyl.

3. The process as claimed in claim 1, wherein alkyl 4-chloroanthranilate (A) is reacted with 4-bromo-2-fluorobenzyl isocyanate (B) to give alkyl N-(4-bromo-2-fluorophenylcarbamoyl)-4-chloroanthranilate (C) and 3-(4-bromo-2-fluorobenzyl)-7-chloro-2,4(1H,3H)-quinazolinedione (D)

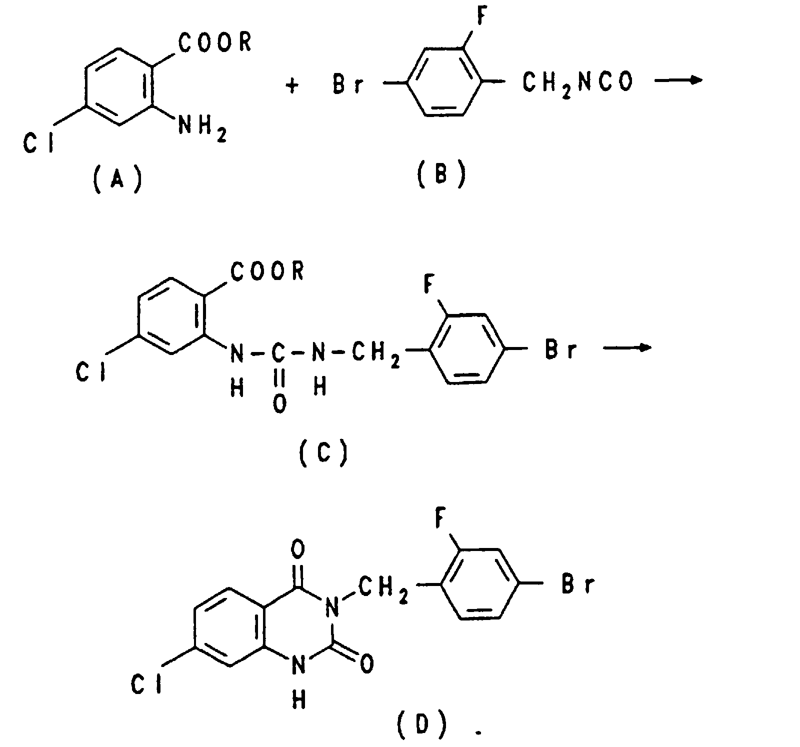

4. The process as claimed in at least one of claims 1 to 3, wherein aromatic, cycloaliphatic or aliphatic hydrocarbons, heterocycles or ketones are employed as the aprotic reaction medium.

5. The process as claimed in at least one of claims 1 to 3, wherein an aromatic hydrocarbon, in particular an alkylbenzene, preferably xylene or a mixture of its isomers, is used as the aprotic reaction medium.

6. The process as claimed in at least one of claims 1 to 5, wherein the alkali metal alcoholates, alkali metal amides, alkali metal hydrides, alkaline earth metal alcoholates, alkaline earth metal amides, alkaline earth metal hydrides or tetraalkylammonium hydroxides are employed in amounts of 1 to 95 mol%, in particular 1 to 20 mol%, preferably 5 to 10 mol%, based on (III).

7. The process as claimed in at least one of claims 1 to 6, wherein sodium methylate in methanol is employed as the alcoholate.

8. The process as claimed in at least one of claims 1 to 7, wherein the reaction of the isocyanate with the anthranilic acid ester is carried out at temperatures from 20 to 150°C, in particular 30 to 120°C.

9. 4-Bromo-2-fluorobenzyl isocyanate.

## Revendications

1. Procédé de préparation de quinazoline-2,4-diones de formule générale (I) 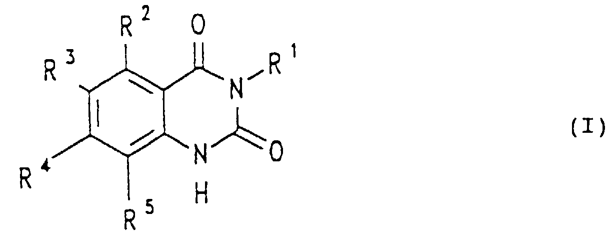 où
R¹ représente des groupes alkyle en C₁-C₁₂, cycloalkyle en C₃-C₈, aralkyle en C₇-C₁₀, le groupe alkyle pouvant être substitué par un ou plusieurs atomes d'halogènes et
R², R³, R⁴, R⁵ représentent, indépendamment les uns des autres, des atomes d'halogènes, des groupes alkyle en C₁-C₃, alkoxy en C₁-C₃ ou des atomes d'hydrogène,
en partant d'un isocyanate R¹-N=C=O et d'un ester de l'acide anthranilique de formule générale (II) 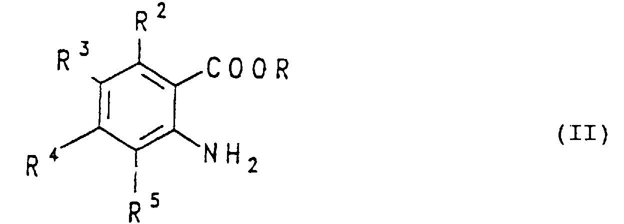 où R est égal à un groupe alkyle en C₁-C₁₀ et R² à R⁵ possèdent les significations données, caractérisé en ce qu'on fait réagir l'ester de l'acide anthranilique dans un milieu réactionnel aprotique sur l'isocyanate R¹-N=C=O pour obtenir des esters de l'acide carbamoyl-anthranilique de formule générale (III) 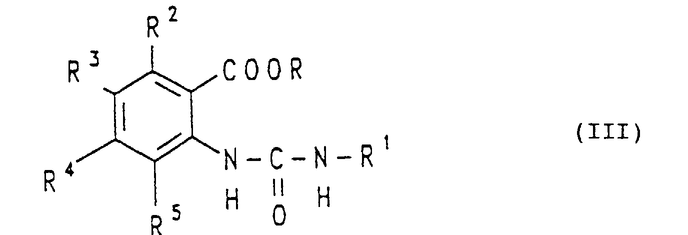 où R, R¹ à R⁵ possèdent les significations données ci-dessus, et on les cyclise en présence d'alcoolates, d'amidures, d'hydrures de métaux alcalins ou alcalino-terreux ou d'hydroxydes de tétraalkylammonium.

2. Procédé selon la revendication 1, caractérisé en ce que R¹ représente des groupes alkyle en C₁-C₆ ou cycloalkyle en C₃-C₆ ou aralkyle en C₇-C₈, les groupes aryle des groupes aralkyle en C₇-C₈ pouvant être substitués de plus par un ou plusieurs atomes d'halogènes, R représente des groupes alkyle en C₁-C₃ et R² à R⁵ représentent, indépendamment les uns des autres, des atomes d'hydrogène, des groupes alkyle en C₁-C₃, alkoxy en C₁-C₃, des atomes de fluor, de chlore ou de brome, en particulier des atomes d'hydrogène, de fluor, de chlore ou des groupes méthyle.

3. Procédé selon la revendication 1, caractérisé en ce qu'on fait réagir des esters de l'acide 4-chloranthranilique (A) sur le 4-bromo-2-fluoro-benzylisocyanate (B) pour obtenir des esters alkyliques de l'acide N-(4-bromo-2-fluorophénylcarbamoyl)-4-chloranthranili que (C) et la 3-(4-bromo-2-fluorobenzyl)--7-chloro-2,4(1H,3H)-quinazoline dione (D).

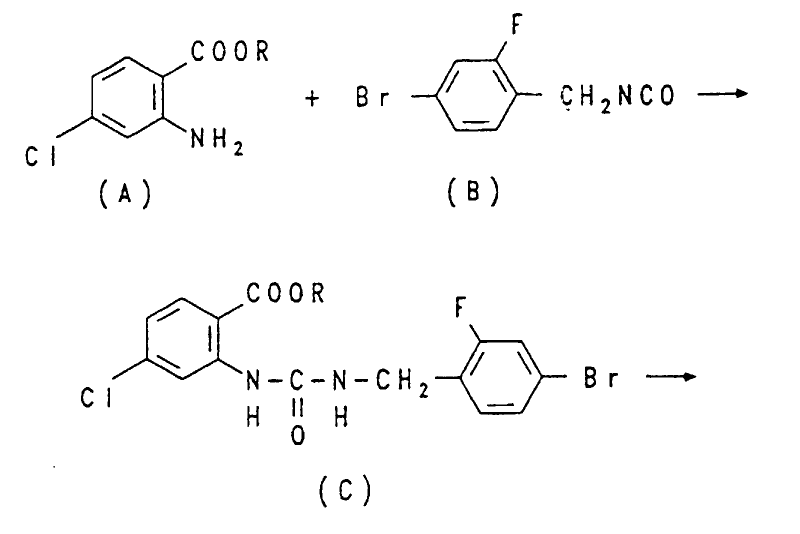

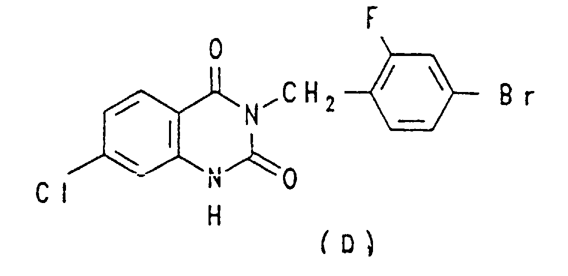

4. Procédé selon au moins l'une des revendications 1 à 3, caractérisé en ce qu'on utilise, en tant que milieu réactionnel aprotique, des hydrocarbures aromatiques, cycloaliphatiques ou aliphatiques, des hétérocyclènes ou des cétones.

5. Procédé selon au moins l'une des revendications 1 à 3, caractérisé en ce qu'on utilise, en tant que milieu réactionnel aprotique, un hydrocarbure aromatique, en particulier un alkylbenzène, de préférence le xylène ou leur mélange d'isomères.

6. Procédé selon au moins l'une des revendications 1 à 5, caractérisé en ce qu'on utilise les alcoolates, les amidures, les hydrures de métaux alcalins ou alcalino-terreux ou les hydroxydes de tétraalkylammonium en quantités de 1 à 95% molaires, en particulier de 1 à 20% molaires, de préférence de 5 à 10% molaires par rapport à (III).

7. Procédé selon au moins l'une des revendications 1 à 6, caractérisé en ce qu'on utilise comme alcoolate le méthylate de sodium dans du méthanol.

8. Procédé selon au moins l'une des revendications 1 à 7, caractérisé en ce qu'on met en oeuvre la réaction de l'isocyanate avec l'ester de l'acide anthranilique à une température de 20 à 150°C, en particulier de 30 à 120°C.

9. 4-Bromo-2-fluorobenzylisocyanate.
